# EUROPEAN PATENT SPECIFICATION

(11) **EP 4 312 764 B1**
(45) Date of publication and mention of the grant of the patent: **25.02.2026**
(21) Application number: 22782348.1
(22) Date of filing: 01.04.2022
(51) Int. Cl.: A61B 5/145, A61B 5/00, A61B 5/1455, A61B 5/11

(54) **MONITORING CHANGES IN VOLUME AND PROPERTIES OF HUMAN INTERSTITIAL FLUID AND ELECTRO-MECHANICAL DESIGN OF AN OPTICAL DEVICE TO ACCOMPLISH THE SAME**
ÜBERWACHUNG VON ÄNDERUNGEN DES VOLUMENS UND DER EIGENSCHAFTEN MENSCHLICHER INTERSTITIELLER FLÜSSIGKEIT UND ELEKTROMECHANISCHES DESIGN EINER OPTISCHEN VORRICHTUNG ZUR DURCHFÜHRUNG DAVON
SURVEILLANCE DE CHANGEMENTS DE VOLUME ET DE PROPRIÉTÉS D'UN LIQUIDE INTERSTITIEL HUMAIN ET CONCEPTION ÉLECTRO-MÉCANIQUE D'UN DISPOSITIF OPTIQUE POUR LA MISE EN OEUVRE DE CELLE-CI

(30) Priority: 01.04.2021 US 202163169557 P
(43) Date of publication of application: 07.02.2024
(73) Proprietor: Bodiguide, Inc., Bellevue, WA 98008 (US)
(72) Inventor: WEKELL, William Oren, Bellevue, Washington 98008 (US)
(74) Representative: Hernandez, Yorck
(86) International application number: PCT/US2022/023174
(87) International publication number: WO 2022/212925

(56) References cited:
- CN-U- 206 167 598
- US-A1- 2004 078 943
- US-A1- 2012 179 067
- US-A1- 2017 290 302
- US-A1- 2019 021 634
- US-A1- 2019 029 566
- US-A1- 2020 237 291
- US-A1- 2020 315 905
- US-B2- 10 136 900

## Description

### BACKGROUND

Fluid balance is a crucial aspect of human physiology. The body automatically makes adjustments to maintain the body's fluid levels under a variety of conditions. Certain medical conditions such as heart failure, kidney disease, and others exceed the body's regulatory mechanisms resulting in excess fluid retention or fluid loading which presents as peripheral edema or limb swelling.

### SUMMARY

The present invention is defined in the appended claims. Fluid retention and edema, or limb swelling, can be associated with and is predictive of an impending decompensation event. Unfortunately, patients do not have good tools for recognizing markers of deteriorating condition. This often forces the patient to the hospital for intervention. This is frightening and expensive.

Measuring and managing fluid balance is associated with many aspects of human health such as heart health, and any number of disease states and medication intervention trials could benefit from an improved wearable device that can detect early warning signs of a new or worsening medical condition in fields that may include, without limitation, nephrology, cardiology, sports medicine, prenatal care, migraines, drug trials, and the like.

### BRIEF DESCRIPTION OF THE DRAWINGS

The detailed description is described with reference to the accompanying figures that illustrate aspects of one or more embodiments described herein, in which the left-most digit(s) of a reference number identifies the figure in which the reference number first appears. The use of the same reference numbers in different figures indicates similar or identical items.
Figure 1 shows an isometric view of an assembled device as an example that can be configured to monitor changes in volume and properties of human interstitial fluid.
Figure 2 shows an exploded view of an example measurement assembly, winder cassette, and strap of a device such as the example illustrated in Figure 1.
Figure 3 is a top view of the assembled device.
Figure 4 is a detail section view through the measurement assembly, winder cassette, and strap of the example device illustrated in Figure 3, taken along line A-A.
Figure 5 shows an isometric view of one optical sensor part, which may correspond to either of the optical sensor parts shown in Figure 4.
Figure 6 shows a breakaway view of winder cassette and strap that remains when the measurement assembly is removed.
Figure 6A shows a breakaway view and a cross section through the winder cassette that remains when the measurement assembly is removed.
Figure 7 shows a view of a strap described with reference to the device illustrated in Figure 2.
Figure 8 shows a plot of normal swelling (e.g., swelling of a limb) over a time period of 12 days for a hypothetical heart failure patient.
Figure 9 shows an 8-day plot of a compensated swelling event (e.g., swelling of an ankle) associated with fluid retention related to increased salt intake for a hypothetical heart failure patient.
Figure 10 shows a plot of a hypothetical decompensated fluid retention episode such as one preceding a heart failure hospitalization.
Figure 11 illustrates an example architecture to implement monitoring changes in volume and properties of human interstitial fluid in a patient.
Figure 12 illustrates various components of a measurement assembly arranged in accordance with one or more embodiments described herein.
Figure 13 illustrates an example of the database server shown in Figure 11.
Figure 14 is a flow diagram of an example process that may be performed at least in part by a measurement assembly for rneasuring limb circumference of a subject, generating a waveform of the measurements over time, and outputting indication of a condition exposed by the waveform.

### DETAILED DESCRIPTION

Interstitial fluid volume is a key medical concern in patients with conditions such as heart failure decompensation, reduced kidney function, and similar physiological conditions. In an example of particular concern, increases in interstitial fluid volume are associated with heart failure decompensation. Taking singular measurements from time to time, perhaps at doctor's appointments, can yield dramatically different and contradictory results from measurement to measurement, depending on the random time of day of the appointment and other factors. Absent presumably more sophisticated measurements, doctors often resort to gross approximation of the presence or absence of swelling, such as pressing their finger into one's ankle, as equivalent in efficacy. Moreover, lacking practical and rigorous medical measurement has constrained the usefulness of this physiological parameter.

Previous approaches to circumference monitoring of limbs have focused on measuring the circumference at a known position on the limb. The device and techniques described herein are not dependent on a known position on the limb but rather take measurements at the minimum circumference regardless of where it occurs as indicating a measure of interstitial fluid volume. The device is constructed to impart only a light tension of the strap around the limb, achievable by design including one or more of lightweight components, the material characteristics of the strap, and the width of the strap. These parameters, as well as the activity of the limb, may all combine to allow the device to come to rest at the minimum circumference of the limb such that the device is continuously sensing the minimum circumference of the limb without unnecessary pressure around the limb at the point of measurement.

The device, in addition to moving along the axis of the limb, can rotate about the axis of the limb. The limb is not circular in cross section. As it clocks about this circumference the area of the winder cassette (600) that bears against the limb has been minimized and the strap (450) can fit more closely to the actual shape of the limb. Thus, the device may allow for consistent measurements regardless of the clocking orientation of the device about the axis of the limb.

A device is described herein that is configured to measure limb circumference and acceleration on an intermittent, periodic, and/or continuous basis, and to analyze the circumference measurements, device and/or subject orientation, and activity data, including comparing the current state or trends with the patient's baseline, normal, or desired state. Differences between measured states and desired states may be used as input to guide decisions about the subject's diet, activity, and treatment, including medication in some implementations, to advance the subject toward the desired state and avoid potentially life-threatening events such as breathless hospitalizations for heart failure patients, in addition to other events such as those described herein.

When the device is used to measure the wrist or ankle volume, it settles at or near the minimum circumference of the limb such that measurements may be compared over time. It may also settle to a repeatable home location because of the construction details. Movement of the limb (for example, the flexing of the Achilles tendon) helps facilitate device mobility on the limb.

One aspect of the device may comprise a low force tensioning element and may be configured to include a large area of distributed load using, e.g., a lightweight strap (in one example, less than 1/2 ounce, inclusive of the device), and articulated or flexible coupling to allow even force distribution. Gravity acting on the mass of the device may contribute force. An additional force is contributed by the mechanism that holds the position device in intimate contact to the limb. For example, tension against the subject's skin exerted by the device, and particularly by the strap under tension, may be less than or substantially equal to (there may be some light compression of the skin) the interstitial fluid pressure at the site and distributed across the surface of the strap such that friction against the skin balances against the force of gravity on the weight of the device. These in conjunction represent the combined forces exerted over a prescribed area. One embodiment of the device may provide a broad surface of approximately 25 millimeters in width disposed around the limb to distribute approximately 11 grams gravitational load applied along the axis of the limb and the constant spring force load of approximately 10-40 grams snugging the strap to the limb.

The disclosed device measures circumference by means of an optoelectronic circuit and a strap encircling the limb. On a portion of the strap is a printed gradient. An optical emitter and detector pair (optical sensor) is situated opposite the gradient. As the limb changes size, the strap expands and contracts via a strap housing and tensioning mechanism which allow the optical sensor to move back and forth across the gradient and detect the amount of reflection associated with a position on the strap which corresponds with a circumference of the limb.

For example, when a person is lying down, interstitial fluid in the body distributes itself somewhat evenly about the length of the body. But when a person is standing or sitting in a relatively vertical position, fluid is redistributed to the limbs due to gravity. This effect is more pronounced in the lower extremities.

In one embodiment of the invention, the device may be worn near continuously and circumference and orientation data are collected at a specified interval and processed to determine a Daily Swelling Pattern and Average Daily Swelling for each individual. The intra day circumference changes related to the redistribution of interstitial fluid from lying to standing is greater than the change in circumference associated with fluid retention of interest associated with health or medical conditions that require attention. Therefore, a plurality of measurements of circumference over circadian cycles are used to detect longitudinal fluid retention.

Careful quantification and tracking over time show that even changes averaging only a few millimeters in fluid volume, which are pertinent physiological information, can present as a significant sign of disease progression but be invisible at a single inspection, or sometimes even daily inspection, and indicate more precise measurement than is possible by visual inspection alone. Capturing a plurality of measurements of minimum limb circumference typical of a narrowing of the limb at an ankle or wrist can be beneficial, as limb circumference can be a close proxy of interstitial fluid volume.

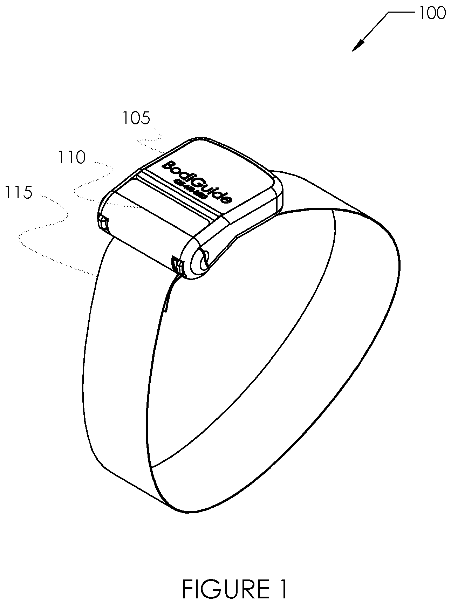

Figure 1 shows an isometric view of an assembled device 100 as an example that can be configured to monitor changes in volume and properties of human interstitial fluid. The device 100 may include, among other components, a device assembly comprising a measurement assembly 105, a winder cassette 110, and a strap 115 that is configured to be placed around the limb of the subject. In at least some embodiments, the subject may wear the device 100 around the lower arm (i.e., below the elbow) or lower leg (i.e., below the knee), although the teachings herein may apply to other body parts, especially body parts that have axial portions, and thus should not be considered limited except by the type of needed data and where the data might be obtained.

The measurement assembly 105 may house an electronics subassembly 225 to obtain data and communicate the data or information based on the data. The electronics subassembly may comprise sensors and associated electronics that, in some embodiments, may perform analysis of sensor data and output results of the analysis and/or recommendations or instructions based on those results. In some embodiments, the electronics subassembly may include a sensor portion comprising a set of optical sensors situated to be opposite a corresponding set of reversed optical gradients on the strap 115, the optical sensors including one or more light emitters and one or more light detectors.

The winder cassette 110 cooperates with the strap 115 generally to enable a loose-tensioned but snug fit to the limb. The device 100 does not ordinarily fit tight to the limb, even in the presence of severe swelling. Indeed, the strap 115 and winder cassette 110 are constructed so that the strap 115 may extend and retract as the limb swells and contracts, enabling an essentially constant force to be applied to the limb.

In some embodiments, the strap 115 is generally flexible and inelastic, and may be made of a biocompatible, porous material for patient comfort. As one example, the material can be .008" Teslin (PPG, Barberton, OH) or Tyvek (Wilmington, DL). It may be brought close to the limb by, e.g., a constant force spring. In one embodiment, the spring may be a 5" long by 5/8" width by .001" half hard stainless steel shim stock (Precision Brands, Downers Grove, IL).

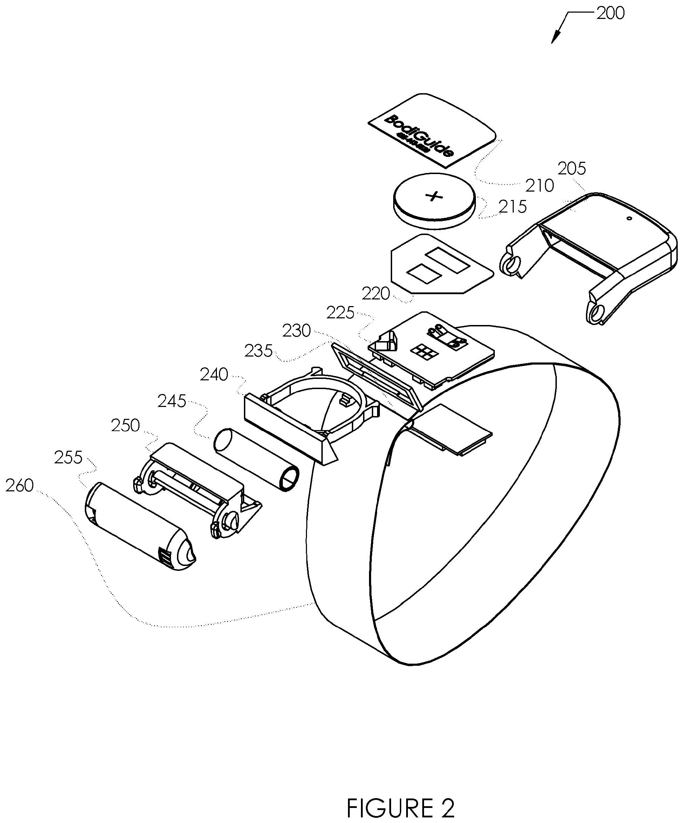

Figure 2 shows an exploded view of an example measurement assembly, winder cassette and strap of a device 200 such as the example illustrated in Figure 1. Figure 2 shows an example that includes an enclosure 205 bearing an optional identification label 210. The enclosure 205 is configured to house one or more of a battery 215, an insulator 220, an electronics subassembly 225 which includes a radio module 230, a gasket 235, and a battery drawer 240 in which to support the battery 215. A spring 245, a spindle 250, and a clasp 255 may form part of the winder cassette 110. The strap 260 may correspond to the strap 115 of Figure 1, respectively. The identification label 210 is shown applied to the enclosure 205, although no limitation should be inferred. An etched label, printed label, or the like may be used. Indeed, the device may have no identification label. The measurement assembly 105 may be substantially fluid-tight to prevent fluid ingress.

The battery 215 may be a so-called coin battery of any sort that meets the power and size requirements of the device 200, and in particular the power suppled to components on the electronics subassembly 225 and other components, and of a size that fits within the battery drawer 240. In some embodiments, another portable power source may used, such as a fuel cell, storage capacitor, energy harvested from the patient, energy harvested from the environment, and the like.

The insulator 220 may be an electrical insulator, and so can be positioned to insulate the battery 215 from the electronics subassembly 225 so that the battery 215 can be changed without contacting any components or wiring on the electronics subassembly 225, or the electronics board substrate. In some embodiments, the insulator 220 may be stamped or printed with configuration information, such as version, for the device 200 or any component thereof.

The electronics subassembly 225 may be a printed circuit board and include electronic components that control and carry out one or more of sensing of limb circumference, running an algorithm on data from the sensed limb circumference, outputting messages, and generating graphic results showing measurements over time that can be interpreted by a medical professional (for example) to give insight into the current condition of a patient wearing the device 200. In some embodiments, the electronic components may include one or more of optoelectronic sensors (including one or more light emitters and light detectors), one or more processors, memory, and an accelerometer. The memory may store instructions that, when executed by the one or more processors, cause the one or more processors to carry out various operations described herein. In at least some embodiments, the accelerometer may detect patient motion and output data that may be used by an on-board control system or transmitted to a remote computing device to determine a level of activity of the patient. For example, the device, by information received from the accelerometer, may detect, accumulate, store, and/or transmit accelerometer measurements at a sufficiently high frequency and associate the information with circumference readings to provide an adequate representation of gravity effects and activity on the limb during the time between circumference measurements. An example of relevant activity is walking. Walking flexes the Achilles tendon and may change the circumferential measurement. If the accelerometer recognizes walking activity, the circumference measurement may be rescheduled or removed from the data set.

The radio module 230 may transmit a digital or analog signal (e.g., containing patient motion information) to an external device. For example, the signal may be transmitted to a nearby computer, smartphone, tablet, or the like which has processing power to receive the signal, analyze the data provided by the signal, and output an instruction or command, relay the signal to a remote computing device such as a server, computer, or database (at a doctor's office or data center, for example).

The gasket 235 is configured and positioned to seal the enclosure 205 and prevent dust or water intrusion that might harm sensitive components inside. The gasket 235 comprises, without limitation, an insulative material suitable for its purpose.

The spring 245 may provide a constant spring force load (for example, approximately 10-40 grams) sufficient to snug the strap 260 to the limb without unnecessary, uncomfortable pressure. The spring 245 may be attached to the strap 260 by an adhesive, for example.

The spindle 250 may be configured to receive the spring 245 at least partially, and provide a place for anchoring and wrapping the spring 245.

The clasp 255 may be configured with cleats so that the strap may be attached by inserting the cleats into corresponding holes in one end of the strap 260. This is similar to holes on a person's belt, as noted above, and allows the device 200 to accommodate a broad range of application from a small wrist to a substantially swollen leg, for example. The sizing of the device may be defined by fixing the length of the strap 200. The holes may be labeled and positioned at a known distance from a known reference point on the gradient at the other end of the strap 260. This sizing data may be collected, stored, and updated as necessary if the "size" is readjusted or calibrated.

The measurement assembly 105 may be constructed such that the insulator 220, electronics subassembly 225 which includes radio module 230, and battery 215, are joined together on the battery drawer 240 with the gasket 235 positioned where the battery drawer 240 meets the enclosure 205 by a snap fit, to facilitate easy removal of the battery drawer assembly for battery 215 replacement. In this configuration, the battery drawer 240 may snap into the enclosure 205, compressing the gasket 235 which seals the interior of the enclosure 205 from dust or water intrusion. Then, the winder cassette 110 may be fitted to the measurement assembly 105, for example by fitting projections on the spindle 250 to corresponding holes or recesses in wings extending from the enclosure 205, bringing together the measurement assembly 105 and the winder cassette 110, including the spring 245, spindle 250, and clasp 255.

In some embodiments, the enclosure 205, spindle 250, and/or clasp 255 may be 3D printed in nylon 12 by a Hewlett Packard multi jet fusion method. These parts may be finished by vapor honing to seal the surface. The battery drawer 240 may have a transparent part facing the optical sensors, and the transparent part may be 3D printed by the Carbon 3D (Redwood City, CA) digital light synthesis process using a Loctite 405 material (Henkel, Germany). The clear part can be sprayed with a Rust-Oleum clear gloss finish (Vernon Hills, IL) to lessen surface diffusion. The gasket 235 may comprise Poron (polyurethane foam).

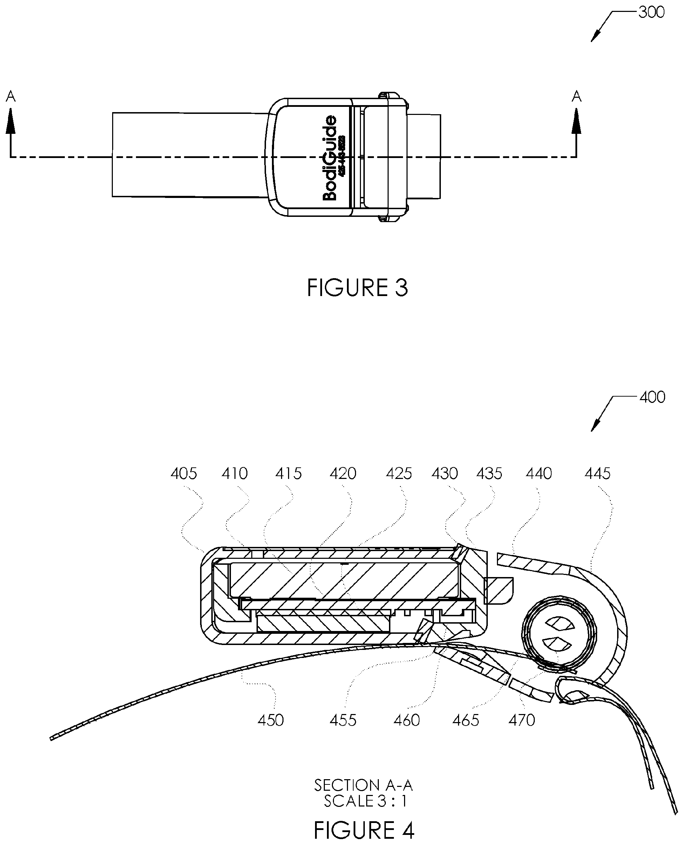

Figure 3 is a top view of the assembled device 300. The device 300 may correspond to the device 100 or the device 200. In practice, the device may drift to its operative, repeatable home location on the limb by the influence of gravity and patient movement, as influenced by friction between the strap 300 and limb.

Figure 4 is a detail section view through the measurement assembly, winder cassette, and strap of the example device illustrated in Figure 3, taken along line A-A. A scale of 3:1 is indicated to give a sense of the size of the device 400, which corresponds to the device 300. However, the scale will change depending on the size of the figure as presented on the page. That is, zooming in or out will affect the scale, and thus 3:1 should not be considered limiting.

In Figure 4, the illustrated components correspond to like-named components in Figure 2. The device 400 may include an enclosure 405, an identity label 410, a battery 415, an electronics subassembly 420 which includes a radio module 425, a gasket 430, a battery drawer 435, a spindle 440, a clasp 445, a strap 450, optical sensor parts 455, and a spring 470. The identity label 410 may be applied to the enclosure 405 as described above. In the illustrated example, the battery 415 may be captured between the electronics subassembly 420 and the top of the enclosure 405, and the radio module 425 portion of the electronics subassembly 420 may bear against the bottom of the enclosure 405. The gasket 430 may be positioned between the enclosure 405 and the battery drawer 435. It is noted that although the sensor devices and strap gradients described herein have a mutual relationship in operation, terms such as "top" and "bottom" should be considered relative and in the case of some electronics components, such relative positions may be interchangeable in practice.

The spindle 440 may be snap-fit into pivot features in the enclosure 405, such as the extensions mentioned above and illustrated in Figure 2. This allows the spindle 440 to rotate and pinch the strap 450 at a point 460 that is opposite the optical sensor parts 455. With this construction, the strap 450, by cooperation with the spring 470, can be in direct contact, or effectively so, with the transparent battery drawer 435 as the strap passes by the battery drawer 435, e.g., when the circumference of the limb increases or decreases. The light path may be at least partially. within the transparent battery drawer. That is, a transparent portion of the battery drawer 435 is located between the optical sensors and gradient portion of the strap, whereby light emitted by the light emitters passes through the transparent portion and reflected by the strap at the gradients back through the transparent portion to the detectors. In some embodiments, the optical sensors may be directly exposed to the gradient without the battery drawer 435 intervening.

In general, the amount of light reflected back toward the optical sensor parts 455 is proportional to the position of the detectors relative to the respective gradient portions. The light sensed by the detectors may be correlated to the position of the device 400 on the limb. This limits tolerance accumulations on parts such as those having an extremely shallow depth of field.

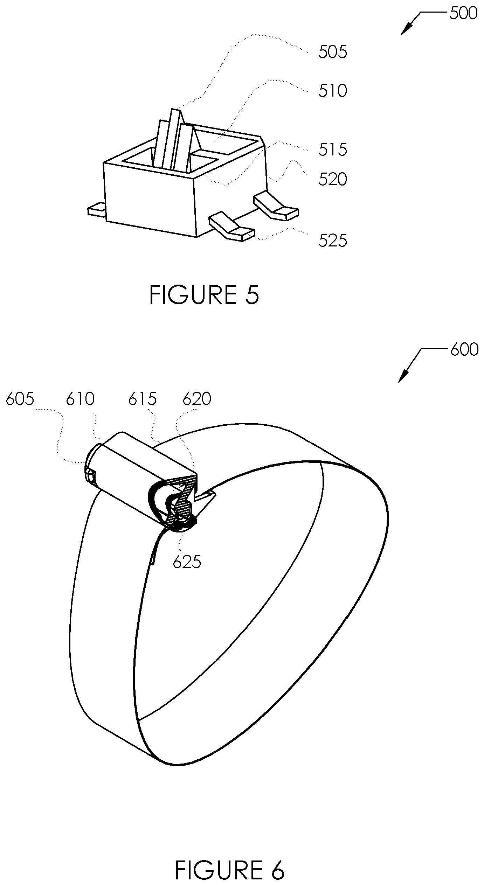

Figure 5 shows an isometric view of one optical sensor part 500, which may correspond to either of the optical sensor parts 455 shown in Figure 4. One optical sensor part 500 is illustrated, although there are at least two optical sensor parts 500 in some embodiments. The illustrated optical sensor part 500 may have conductive legs configured to be soldered to electrical contacts on the electronics subassembly 420 or otherwise operably associated with the electronics subassembly 420 by, e.g., mounting to a socket that is in electrical contact with the electronics subassembly 420. Further, the optical sensor part 500 has an enclosure 520 that contains an emitter 515 and a detector 510 in some embodiments. The position of the illustrated coupling 505 or focal distance is shown.

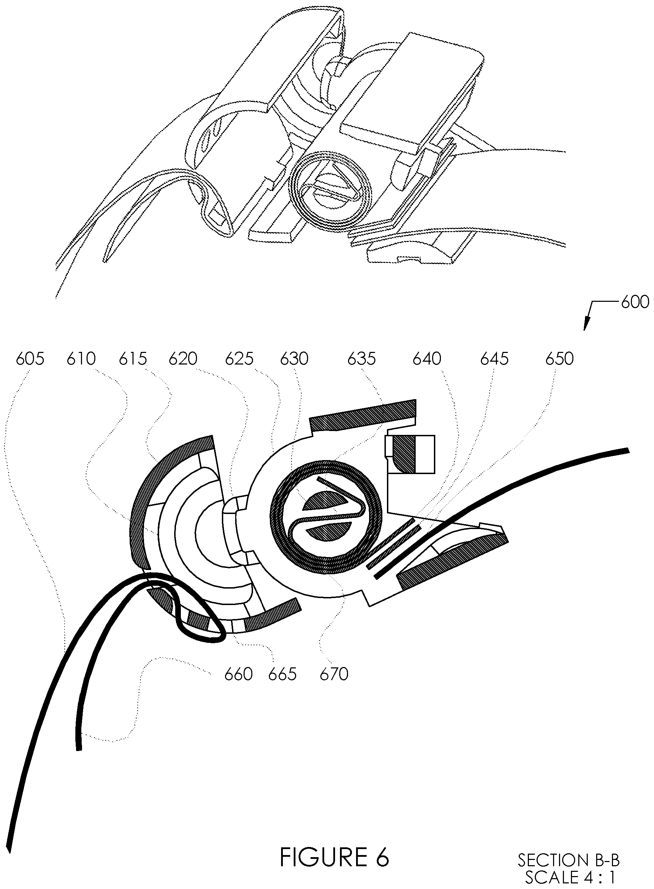

Figure 6 shows a breakaway view of winder cassette 600 and strap 615 that remains when the measurement assembly is removed. The winder cassette 600 may correspond to the winder cassette 110 illustrated in Figure 1, for example. The winder cassette 600 may comprise a clasp 605 and a spindle assembly comprising a spindle 610 and a spring 620 that may correspond to the clasp 255, spindle 250, and spring 245 shown in Figure 2. The clasp 605 is shown joined to the spindle 610 at a snap latch 625. In some embodiments, the winder cassette 600 combined with strap 615 may be replaceable.

The action of moving the gradient on the strap 615 past the optical sensor(s) as the limb expands and contracts may be accomplished with a tensioning mechanism comprising the spindle 610 and spring 620 within the spindle assembly that may house a portion of the length of the strap 615 that is rolled and attached to the spring 620. In some embodiments, the spring 620 may be a constant tension spring. As the limb expands, the strap 615 unrolls, increasing the length of the strap 615 to accommodate the increased circumference of the limb while maintaining a constant tension of the strap 615 around the limb by a constant force applied by the spring 620. The tension of the strap 615 may be matched to the interstitial fluid pressure and elasticity of the skin such that the device expands and contracts without creating an indentation in the limb. In this regard, and in conjunction with the other embodiments described herein, it is understood that constancy of the force and tension need not be exact, but are within a reasonable tolerance that enables the device to perform its function of measuring limb circumference, and particularly differences thereof relative to a baseline or other reference, in accordance with the principles outlined in this disclosure.

Limb circumference measurements and limb orientation data are taken continuously at a regular interval and processed to produce a personal Daily Swelling Pattern for the subject wearing the device. The Daily Swelling Pattern is characterized by a minimum limb circumference that occurs when the subject is lying down and at a maximum circumference after the subject has been in a vertical position such as standing or sitting for a period of time specific to that individual.

Trends in the fluid gain or loss are computed for specified time periods such as days, weeks, or months.

Fluid gain/loss and fluid gain/loss trends can be compared to threshold values to identify conditions of interest. The system takes actions specific to the condition of interest including sending messages and alerts to the user as well as support personnel such as family caregivers, chronic care management, and/or clinical personnel.

The rate at which fluid redistributes itself in the body on rising to a vertical orientation may be indicative of the viscosity of the interstitial fluid; changes in viscosity are known to be related to heart failure decompensation due to changes in protein levels in the interstitial fluid. This is typically evaluated by a physician pushing a finger firmly against the ankle of the patient and seeing if the "dent" produced rebounds quickly. If the dent is slow to rebound, the condition is described as pitting edema. This is a significant medical sign and useful diagnostic method in characterizing the condition of the patient.

The disclosed techniques can characterize the rate of change of the redistribution of interstitial fluid. By taking a plurality of measurements when the patient moves from a supine to upright orientation, typically in the morning, it is possible to track the time it takes for the redistribution of interstitial fluid associated with the change in direction of the gravitational force. This rate of change measurement is directly related to the viscosity of the interstitial fluid. Being able to recognize interstitial fluid viscosity and changes associated with it can further inform the medical practitioner or computed algorithm about changes in the disease state of the patient, as fluid viscosity offers insight to the underlying cause of fluid loading (e.g., change in protein levels in the interstitial fluid).

The measurement device accommodates variability of limb size in two ways. As the limb changes in size, the strap 260 may move past the optoelectronic sensors on the electronics subassembly 225. At the other end of the strap 260, the clasp 255 may be joined by cleats to the strap 260 at one of a plurality of hole features that define the gross overall size of limb the assembly can accommodate.

Figure 6A shows a breakaway view and a cross section through the winder cassette 600 that remains when the measurement assembly is removed. The winder cassette 600 may correspond to the winder cassette 110 illustrated in Figure 1, for example. The winder cassette 600 may comprise a clasp 615 and spindle 625. In some embodiments, the spindle 625 may be hollow. In such embodiments, inside the spindle 625 may be a wrapped or coiled spring 670 attached to a capture feature 630 fixed to the inside of the spindle 615. The coil spring 670 may have a narrow wire or broad band-like construction, for example. In some examples, the capture feature 630 may comprise first and second pin portions fixed within the spindle 615, such as within one or more grooves or holes in the spindle walls to receive corresponding ends of the pin portions, or extensions from one or both spindle walls on which to mount hollow end portions of the pin portions, for example. In some embodiments, one or both ends of the pin portions may be extruded from or fixed to the spindle wall or walls.

The spring 670 may be attached to the capture feature 630 by, e.g., inserting one end of the spring 670 into a gap between the first and second pin portions and wrapping or coiling the spring 670 around the first and second pin portions as a set. To aid with fixing the spring 670 to the spindle 625, the end of the spring 670 that is inserted into the gap may be wrapped or bent to wrap at least partially around one of the pin portions. Bends 635 in the spring 670 may engage the spindle capture feature 630. as shown in Figure 6. The distal end 640 of the spring 670 may be j oined by adhesive 645 to the proximal end 650 of the strap 605, although no limitation on a particular type of fixing should be inferred.

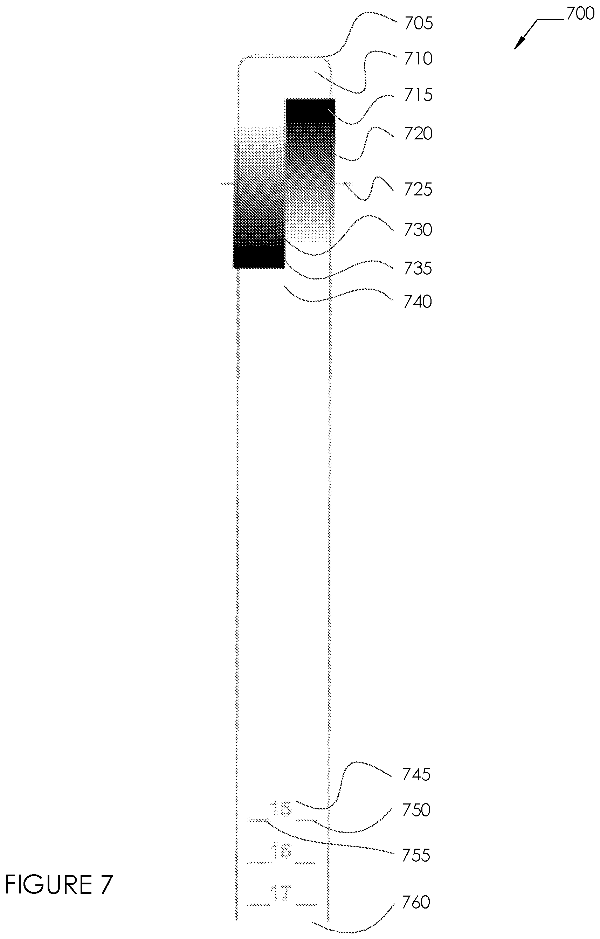

The clasp 615 can be joined to the spindle 625 by flexible latch features 620 that are integral to the spindle 625. These latch features engage geometry 610 in the clasp 615 to join the ends of the winder cassette 600 in order to encircle the limb. The distal end 660 of the strap 605 is threaded through the body of the clasp 615 and secured by one or more cleats 665 to one of a plurality of receiving features 750 in the strap 700 as shown in Figure 7. In some embodiments, the winder cassette 600 combined with strap 605 is a replaceable component that a user can change out easily if it were to become soiled or broken.

Figure 7 shows a view of a strap 700 described with reference to the device 200 illustrated in Figure 2. The strap 700 may correspond to at least the strap 260, for example. Near one end 705 of the strap 700 is a blank area 710 which is no larger than a length needed to adhere the strap 700 to the constant force spring 245.

In one example, the strap 700 may be positioned such that two of the optical devices 520 shown in Figure 5 are positioned over gradient areas 720, 730 at a nominal position 725. In some embodiments, the optical sensors 520. In some embodiments, the end of the strap 700 having the optical gradients 720, 730 may be positioned to pass beneath the optical sensors 520. Wherever the device settles on the patient's limb, the device can be calibrated when the calculated value for the detector output is within a predetermined range when adjacent the end zone of one gradient. If the calculated value for the detector is not within the predetermined range, an on-board or remote processor may determine that a bad read has occurred. On the other hand, if the calculated value for the detector is within the predetermined range, the processor may determine that a good read has occurred.

The gradients 720, 730 may range from dark to light, and be arranged 180 degrees to each other as in the example shown. Further, at the end of each gradient 720 and 730 may be an "end zone" area 715 and 735, respectively, of the darkest part of the gradient, which ensures signal continuity under large excursions. Correspondingly, there may be an optical "home zone" area 740 beyond the ends of both gradients 720, 730 where both optical devices 520 may be exposed to white, or to an unprinted area or area that is at least substantially lighter than the end zone of the gradient 730. Similarly, bands of known reflectance may be positioned adjacent to at least one of the gradients to enable the optical sensor(s) and downstream signal and data processing to recognize operational events (e.g., out of bounds, fully retracted, fully expanded, resizing, and/or the like).

For example, the end zones may be solid or nearly solid black. The end zones thus represent an out-of-range condition; e.g., when the optical sensors 520 sense the end zone or a difference between relatively very dark to relatively very light, for example by the difference in determined light amplitudes exceeding a certain threshold. In some embodiments, the sensed regions are represented by a preset light amplitude detectable by the optical detectors 520, respectively. In this condition, one of the optical devices 520 is exposed to black (or a portion identifiable as the end zone) and the other white (or a portion identifiable as the opposing relatively light zone). This allows for messaging in response to the detection to refit the clasp 255 (generally manually) to a smaller or larger cleat position.

As indicated, in some examples, two optical sensors 520 may be situated opposite the reversed gradients 720, 730. This configuration generates a complementary set of data such that signals can be combined (added) in a manner that errors related to the nonlinearity of the transducers are offset and thereby produce a more accurate result of position. In particular, the amplitudes of light reflected from corresponding locations of one gradient that gets darker in one direction and the other gradient that gets lighter in the same direction will have detectable equal-and-opposite nonlinearities that will cancel and extend the linear operating range. Similarly, any error that affects both channels in a common way such as ambient light, temperature, or voltage may be reduced analogously to common mode rejection techniques in electronic circuits. Alternatively, two optical sensors positioned over a single gradient can be combined to achieve the same effect by subtracting their signals. An additional or alternate construction is to design a gradient that compensates for the nonlinearity of the optical sensors.

An additional or alternate construction is to situate the optical sensors 520 opposite the same gradient. In such embodiments, knowing the gradation of reflectivity (light-to-dark and vice versa) over specific measuring points enables interpolation between sensor outputs to determine expansion and contraction of the strap 700 due to limb swelling.

The sensitivity for the optical sensors 520 may vary, which may result in a variable reflectance operating range for each optical sensor 520 when used as a position sensor. A gradient operating range may be calibrated based at least on the specific sensitivity of each optical sensor 520. For example, in at least one embodiment, a digital pot can be added to the emitter and detector circuit such that the circuit is modified as part of the optical sensor calibration to achieve multiple operational benefits including obtaining a full operating range of reflectance, improved linearity of the optical sensor, and use of a common gradient for all sensors.

Using an optical sensor 520 as a position sensor may include maintaining a fixed distance between the optical sensor 520 and the reflective surface of its associated gradient. This can be accomplished by stabilizing the height of the optical sensor 520 within the case body using the transparent battery drawer containing the battery (and electronics subassembly in some embodiments). Consider, for example, the arrangement shown in Figure 4. The strap containing the gradient at 460 may be held firmly against the external surface of the transparent battery drawer 435 by a rocker pinch mechanism incorporated into the winder cassette that acts as a spring when the winder cassette is mated to the measurement assembly.

In some embodiments, when the device is not being worn, the spring 245 may retract the strap 700 to the home zone or beyond, which can be read by the software that is executed to interpret the signals representing the detect light amplitudes, with an indicator or message output informing the same via a display on the enclosure. In some embodiments, the output may be a signal transmitted to another device to provide the information to the receiving device. The detection of these conditions can also be used to indicate whether a strap needs to be resized larger or smaller.

At the distal end 760 of the strap 700 are a series of cut features 750, 755 that engage the clasp cleats of the clasp 255 to set the usable circumference of the assembly. As indicated elsewhere, the cut features 750, 755 may be considered to be analogous to holes in a belt. There is a number 745 associated with each of the cut features 750, 755 that represents, for example, the number of centimeters from the nominal middle of the gradients 720, 730, respectively. Straps 700 of various lengths are possible for small and large limbs but all use the same or similar principles of construction and may be trimmed of objectionable overage.

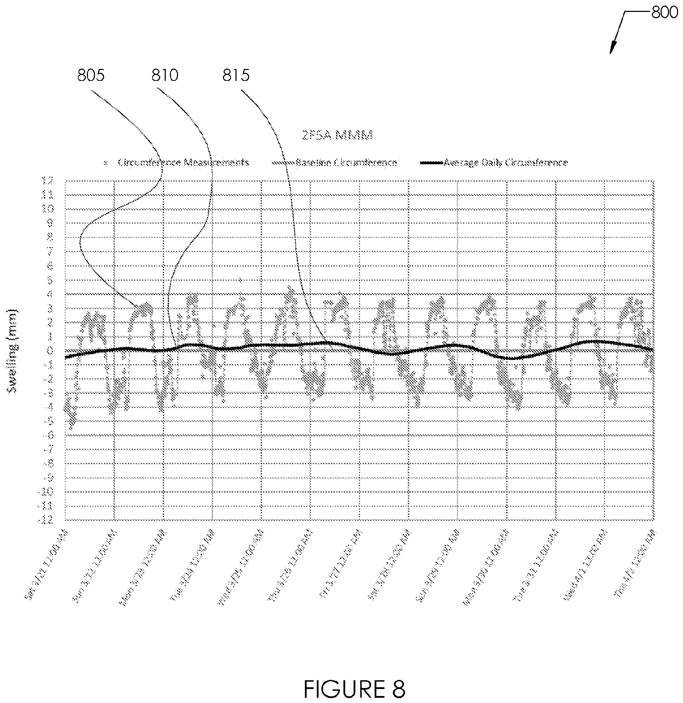

Figure 8 shows a plot 800 of normal swelling (e.g., swelling of a limb) over a time period of 12 days for a hypothetical heart failure patient. The reference number 805 is associated with a plot of the circumference readings captured by the device (e.g., device 100) vs. time and shows the repetitive Daily Swelling Pattern over the 12 days. The reference number 810 is associated with the rolling average of the ankle circumference. In some embodiments, an exponential moving average may be used. The reference number 815 is associated with a user's normal baseline circumference that is derived from circumference readings measured during a user's normal or "dry" state in the example of the heart failure patient.

A swelling pattern can be represented by an "average" circumference considering the minimum and maximum circumferences associated with the swelling pattern.

The Baseline Swelling Pattern or the Baseline Average Swelling is identified as the Daily Swelling Pattern or Average Daily Swelling detected when the subject is in a normal state of health usually referred to as a "dry" state for heart failure patients. The systems can compute or adjust the normal baseline over a period of use. The system maintains the normal baseline for comparisons to compute fluid gain/loss.

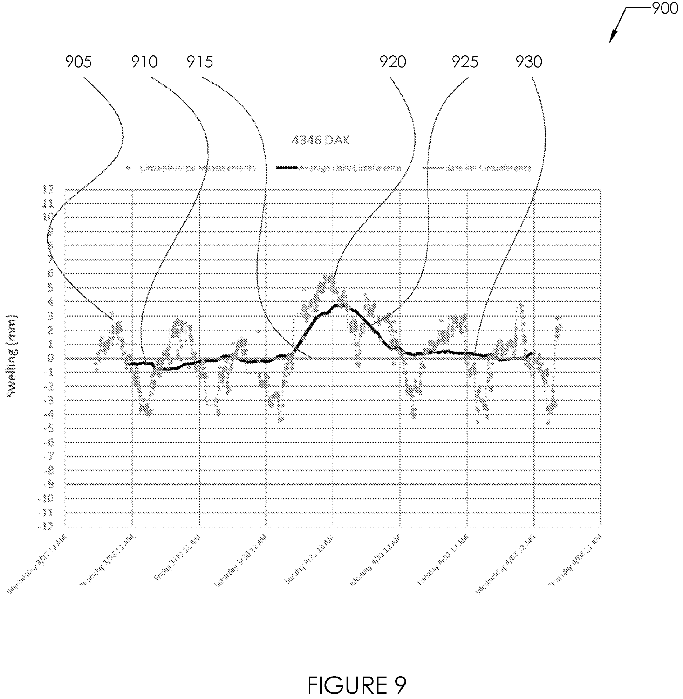

Figure 9 shows an 8-day plot 900 of a compensated swelling event (e.g., swelling of an ankle) associated with fluid retention related to increased salt intake for a hypothetical heart failure patient. The reference number 905 is associated with a plot of the circumference readings captured by the device (e.g., device 100) vs. time. The reference number 910 is associated with the rolling average of the ankle circumference. A similar plot can be made if using an exponential moving average. The reference number 915 is associated with the patient's normal baseline circumference that is derived from circumference readings, e.g. circumference readings measured during a user's normal or "dry" state in the case of the heart failure patient. The reference number 920 shows the deviation in the Daily Swelling Pattern that may be associated with fluid retention for this patient, in this case intake of high-salt meals for two consecutive days. The reference number 925 points to the corresponding change in average circumference that occurs during the swelling event. The reference number 930 points to the return to normal swelling in the days following the event as the patient's body compensates and eliminates the extra fluid.

In Figure 9 there are quite different waveform plots of individual measurements (905). These patterns are different between individuals and days. And this individual's pattern is substantially less regular than the previous example. But it is clear from the moving average trend line (925) that a 4 millimeter increase in swelling occurred over a 2 day period. This coincided with a heavily salty meal. The swelling subsided (930) over time as the body compensated for this infusion.

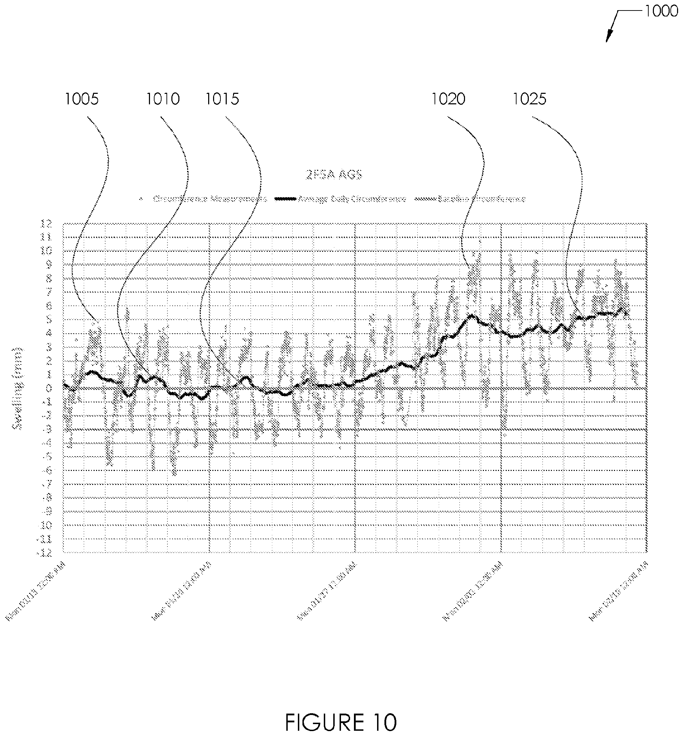

Figure 10 shows a plot 1000 of a hypothetical decompensated fluid retention episode such as one preceding a heart failure hospitalization. The reference number 1005 is associated with a plot of the circumference readings captured by the device (e.g., device 100) vs. time over four weeks. The reference number 1010 is associated with the rolling average of the ankle circumference. A similar plot can be made using an exponential moving average. The reference number 1015 is associated with the patient's normal baseline circumference that is derived from circumference readings measured during a user's normal or "dry" state in the case of heart failure patients. The reference number 1020 shows the deviation in Daily Swelling Pattern that may be associated with fluid retention, in this case a trend associated with decompensation related to a condition that requires clinical intervention. The reference number 1025 is the corresponding change in average circumference associated with increasing fluid retention. The reference number 1030 points to the return to normal swelling in the days following the event as the patient's body compensates and eliminates the extra fluid.

The plot 1000 of Figure 10 shows yet a different pattern of measurements. The excursions in circumference measurement can exceed 10 millimeters over a day while the Average Daily Swelling signal change of several millimeters is the signal of interest. The potential error, deviating from the arithmetically processed measurements, is larger than the signal for a single measurement. This patient had a much larger daily range of measurements (1005) as well as a substantial increase in monthly excursions. On essentially the same location on the same ankle, there is a swelling range of 17 millimeters over the month. Of interest is that, during the first two-week period, the average swelling remains in a plus or minus 1 millimeter range (1010). However, during the subsequent two-week period, the Average Daily Swelling increases to almost 6 millimeters (1025) above the nominal baseline(1015). This represents a substantial and persistent trending of the circumference data indicative of increases in patient fluid volume.

This persistent trending can be compared with the individual patient's history as well as patients who might have similar characteristics such as, but not limited to, age, height, weight, left ventricle ejection fraction, comorbidities, and/or similar measures. Additionally, other physiological measures such as, but not limited to, heart rate, SpO2, NIBP, and/or heart rate variability may also be considered in conjunction with these measurements. Processing this information, using appropriate correlative algorithms, may enable a real-time, consistent, continuous, and/or instant acute evaluation of the trending event severity and predict the likelihood of an impending decompensation event. In some embodiments, trained machine learning models or rules application algorithms may be utilized and updated in accordance with feedback (human or machine) from the patient's experience and/or from a population of patients to constantly improve the accuracy of these predictions; the more measurements taken; the more accurate the predictions, especially in order to minimize interpolation errors and determine an accurate model of changes of daily swelling of a limb. When a substantial swelling event exceeding a predetermined threshold stored on the device or remotely is detected, a patient or their caregiver may be notified to take actions to change overall patient activity, contact their medical provider, change the amount of a treatment such as a diuretic, add or subtract other medications, or implement other methods that may alter the course of the disease. In some examples, the responsive actions are urgently provided; in others, such as diet change, the responsive actions may be suggestions or instructions. Changes in medication dosage would normally follow a predefined treatment plan from the patient's physician, where an additional diuretic might be indicated when the patient has a substantial swelling event, for example.

Given the small size of the arithmetically processed signal of interest compared to the range of circumference measurements over a day, it is desirable to minimize sampling rate induced distortions of the underlying, continuous changing, physiology. Linear interpolations that truncate the actual limb swelling excursion values can materially change the value of the calculated signal of interest. In the simplest of examples, a plot of circumference measurement at a random time during a week could yield substantially different results that might substantively contradict a more densely sampled identical patient and condition. This is particularly true when evaluating the rate of change of circumference associated with changes in gravitational orientation of the patient.

In the nonlimiting examples shown in Figures 8, 9, and 10, measurements are taken every 10 minutes. But it is understood that different intervals are within the scope of this disclosure.

These individual measurements may be mathematically aggregated over a period of time, such as a day, 48 hours, or any time suited to the monitoring and analysis. In this example it is by means of a rolling average; an exponential moving average is also contemplated. A series of these aggregated measurements are then compared for evidence of deviation or divergence. In the example of Figure 8, for example, there are a series of points representing individual circumference measurements. It should be noted that there is a fair amount of variability in these measurements.

Measurements for this individual ranged 10 millimeters for the same position on the same ankle depending on the day and time. But the aggregate daily oscillation in measurements is in fact quite stable. The range for this individual was about a millimeter as can be seen in the average daily circumference trend line (815) .

This paper describes a method of capturing a plurality of measurements of minimum limb circumference typical of a narrowing of the limb at an ankle or wrist, for example; it should be noted that the teachings herein are applicable whether the minimum limb circumference is at a narrow or narrowing point or at a location on a constant circumference, such as a constant cylinder. And limb circumference is a close proximate of interstitial fluid volume. And interstitial fluid volume is a key medical concern in patients with conditions such as heart failure decompensation, kidney function and similar physiological conditions. Increases in interstitial fluid volume are associated with heart failure decompensation. It should be clear from Figures 8, 9 and 10 that a single measurement, perhaps at a doctor's appointment, would yield dramatically different and contradictory results depending on the random time of day of the appointment. This has limited doctors to gross approximation of the presence or absence of swelling, such as pressing their finger into your ankle. Lacking practical and rigorous medical measurement has constrained the usefulness of this physiological parameter. It is with the careful quantification and tracking over time that changes in fluid volume, that are pertinent physiological information, are apparent. Average changes of a few millimeters can present as a significant sign of disease progression but are invisible at a single inspection, or daily inspection, and require more precise measurement than is possible by visual inspection alone.

It should be noted that a single measurement at a fixed time of day would not yield accurate results. As can be seen in Figures 8, 9, and 10, extreme excursions of the measurement do not necessarily occur at the same time each day.

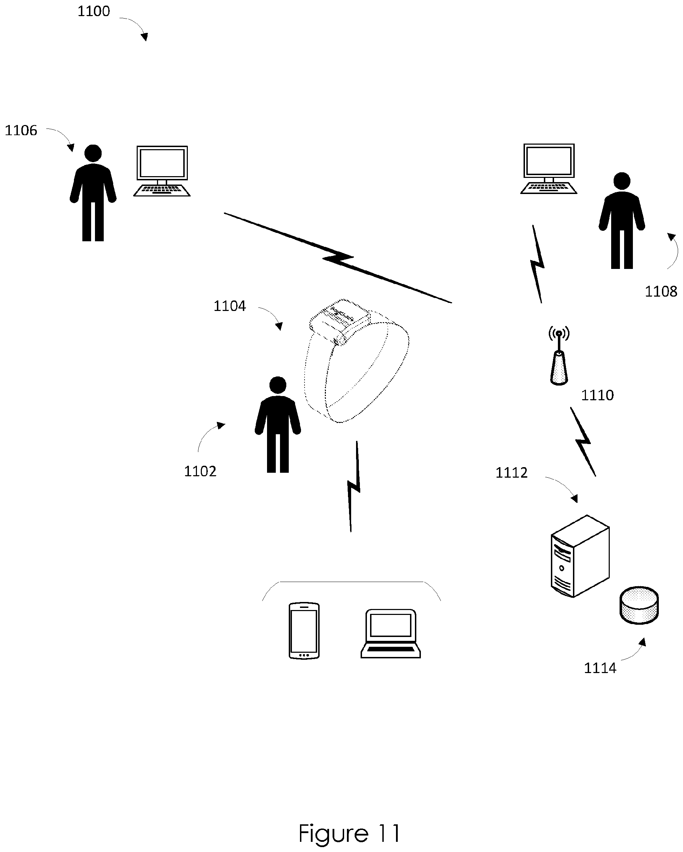

Figure 11 illustrates an example architecture 1100 to implement monitoring changes in volume and properties of human interstitial fluid in a patient 1102. The illustrated architecture 1100 includes a device 1104, a health care entity 1106, a personal contact 1108, and a wireless access point 1110, although this is an example only and other configurations including more components or fewer are contemplated.

The health care entity 1106 may include, without limitation, a medical facility, caregiver, and/or other personnel associated with patient care A caregiver or other personnel may operate one or more computing devices as part of their care function.

The personal contact 1108 may include support personnel operating one or more computing devices connected to a database server 1114 via a wired or wireless communication link (e.g., the Internet or other wireless and/or wired connection) using SMS messages, WIFI protocols, Bluetooth protocols, and the like The personal contact 1108 may also include a personal representative for the patient, family member, or other individual set up to receive information derived from the device 100.

A control system may include a database server 1112 connected to a database 1114. The database 120 may store pertinent data about the patient 102 (such as patient history, a patient record, and the like), trigger event levels, and addresses to which messages (e.g., notifications, alerts, and the like) are to be sent.

Information from the device 1104 can travel several alternate paths depending upon the implementation details. For example, the information may be input into a computing device (e.g., a patient desktop computer, a patient cellular telephone, a patient portable computer, and the like) connected to the database server 1112 (or a web server) via the Internet or other network The computing device may transfer the feedback information to the database server 1112 directly or via the access point 1110. The device 1104 may communicate the device messages to the computing device for transmission thereby to the database server 1112.

The device 100 may continuously measure and store position measurements on the device itself or remotely, e.g., at the database 1114, at a predefined frequency. The positions may be interpreted as a relative circumference measurement when compared to an arbitrary reference or an absolute circumference measurement when combined with the size information which establishes a relationship between a position on the gradient and a known circumference.

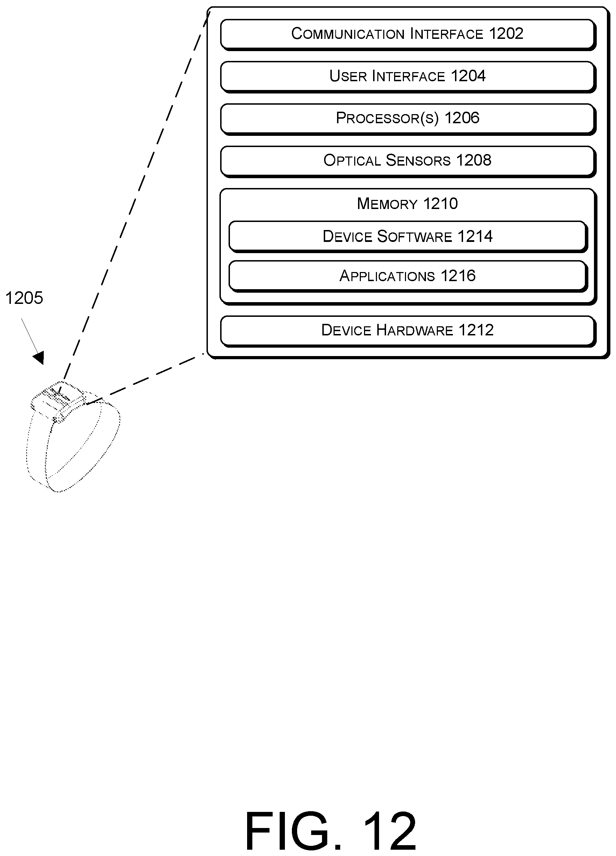

Figure 12 illustrates various components of a measurement assembly 1205 arranged in accordance with one or more embodiments described herein. The measurement assembly 1205 may correspond to the measurement assembly 105 shown in Figure 1, for example. In the illustrated example, the measurement assembly 1205 may be configured to measure the circumference of a limb using one or more sensors, such as optical sensors, by detecting the extension or retraction of a strap attached to a winder cassette coupled to the measurement assembly.

As illustrated in Figure 12, the measurement assembly 1205 may include one or more of a communication interface 1202, a user interface 1204, one or more processors 1206, one or more optical sensors 1208, memory 1210, and device hardware 1212.

The communication interface 1202 may include wireless and/or wired communication components that enable the measurement assembly 1205 to transmit data to and receive data from other networked devices via a communication network such as that described with respect to Figure 11.

The user interface 1204 may enable a user to provide input and receive output from the measurement assembly 1205, including for example providing one or more input to initiate device activation and/or set metadata, tags, communication parameters, monitoring parameters, etc. The user interface 1204 may include a data output device (e.g., visual display, audio speakers), and one or more data input devices. The data input devices may include, but are not limited to, combinations of one or more of touch screens, physical buttons, cameras, fingerprint readers, keypads, keyboards, mouse devices, microphones, speech recognition packages, and any other suitable devices or other electronic/software selection methods.

The processor(s) 1206 and the memory 1210 may implement an operating system. The operating system may include components that enable the measurement assembly 1205 to receive and transmit data via various interfaces (e.g., the user interface 1204, the communication interface 1202, and/or memory input/output devices), as well as process data using the processor(s) 1206 to generate output. The operating system may include a display component that presents output (e.g., displays data on an electronic display, store the data in memory, transmit the data to another electronic device, etc.). Additionally, the operating system may include other components that perform various additional functions generally associated with an operating system.

The optical sensors 1208 may be configured by light-emitting and/or - detecting devices (for example, optical emitter/detector pairs) sized to fit within the enclosure of a wearable device of the type described herein, working in conjunction with optoelectronic circuitry to detect the amount of reflection associated with a position on the strap which corresponds with a circumference of the limb.

The memory 1210 may be implemented using computer-readable media, such as computer storage media. Computer-readable media includes, at least, two types of computer-readable media, namely computer storage media and communications media. Computer storage media includes volatile and non-volatile, removable and non-removable media implemented in any method or technology for storage of information such as computer-readable instructions, data structures, program modules, or other data. Computer storage media includes, but is not limited to, Random-Access Memory (RAM), Dynamic Random-Access Memory (DRAM), Read-Only Memory (ROM), Electrically Erasable Programable Read-Only Memory (EEPROM), flash memory or other memory technology, CD-ROM, digital versatile disks (DVD) or other optical storage, magnetic cassettes, magnetic tape, magnetic disk storage or other magnetic storage devices, or any other non-transmission medium that can be used to store information for access by a computing device. Computer readable storage media do not consist of, and are not formed exclusively by, modulated data signals, such as a carrier wave. In contrast, communication media may embody computer-readable instructions, data structures, program modules, or other data in a modulated data signal, such as a carrier wave, or other transmission mechanism.

The memory 1210 may include the operating system, device software 1214, and one or more applications 1216, as well as data gathered by the optical sensors or input by a user or received from a remote source. The application(s) 1216 may include any application software executable by the one or more processors 1206, including but not limited to applications that facilitate the functions of the wearable device 1205 for detecting extension and retraction of the strap by detecting and processing reflected light and data derived therefrom; manipulation, formatting, addition, deletion, or modification of metadata; and image or data processing, for example.

The device software 1216 may include software components that enable the measurement assembly 1205 to perform functions. For example, the device software 1216 may include a basic input/output system (BIOS), Boot ROM, or bootloader that boots up the measurement assembly 1205 and executes the operating system following power up of the measurement assembly.

The device hardware 1212 may include additional hardware that facilitates performance of the user interface 1204, data display, data communication, data storage, and/or other device functions.

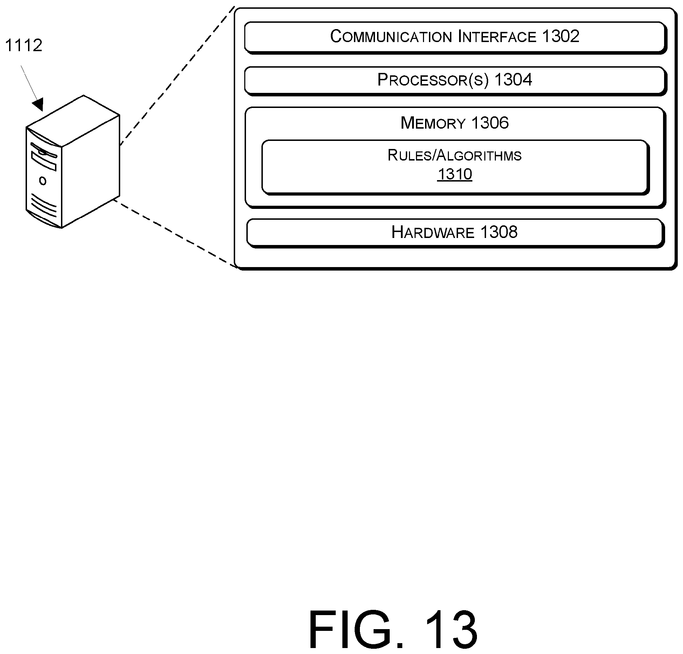

Figure 13 illustrates an example of the database server 1112 shown in Figure 11. The database server 1112 may include a communication interface 1302, one or more processors 1304, memory 1306, and hardware 1308. The communication interface 1302, like the communication interface 1202 of the measurement assembly 1205, may include wireless and/or wired communication components that enable the database server 1112 to transmit data to and receive data from the measurement assembly 1205 and other networked devices via a communication network such as that described with respect to Figure 11.

The processor(s) 1304 and the memory 1306 may implement an operating system. The operating system may include components that enable the database server 1112 to receive and transmit data via various interfaces (e.g., the communication interface 1302 and/or memory input/output devices), as well as process data using the processor(s) 1304 to generate output. The operating system may include a display component that presents output (e.g., displays data on an electronic display, store the data in memory, transmit the data to another electronic device, etc.). Additionally, the operating system may include other components that perform various additional functions generally associated with an operating system.

The memory 1306 may include the operating system, device software, and one or more applications, as well as data gathered by the optical sensors or input by a user or received from a remote source. The applications may include any application software executable by the one or more processors 1304, including but not limited to applications that train and/or execute rules or machine learning models and algorithms 1310 to process data received from the measurement assembly 1205, including generating waveforms of circumference changes, interpreting data, applying activity information, subject medical history, and other data, generating predictive output for analysis and/or feeding back to update or retrain models, and the like.

The hardware 1308 may include additional hardware that facilitates performance of data display, data communication, data storage, and/or other device functions.

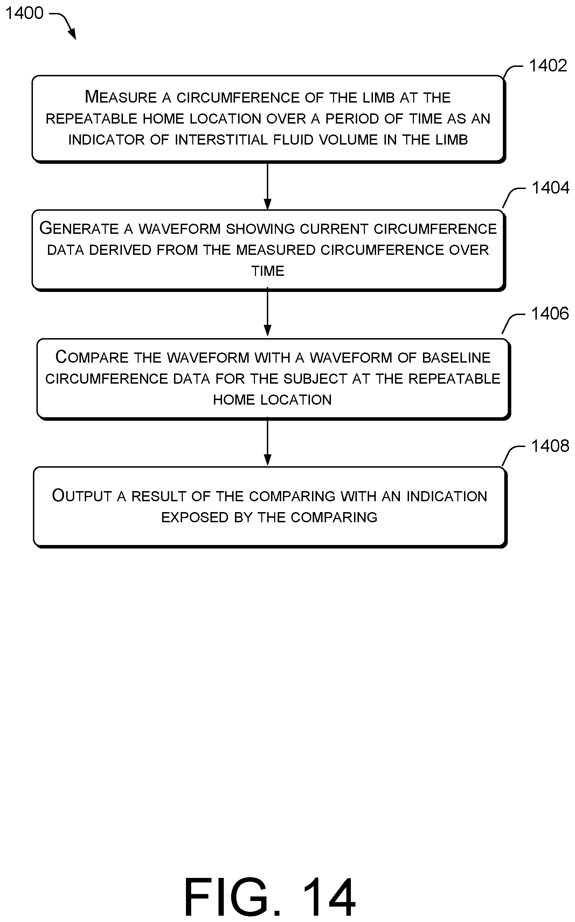

Figure 14 is a flow diagram of an example process 1400 that may be performed at least in part by the measurement assembly 105 for measuring limb circumference of a subject, generating a waveform of the measurements over time, and outputting indication of a condition exposed by the waveform. The process 1400 is illustrated as a collection of blocks in a logical flow chart, which represents a sequence of operations that can be implemented in hardware, software, or a combination thereof. In the context of software, the blocks represent computer-executable instructions that, when executed by one or more processors, perform the recited operations. Generally, computer-executable instructions may include routines, programs, objects, components, data structures, and the like that perform particular functions or implement particular abstract data types. The order in which the operations are described is not intended to be construed as a limitation, and any number of the described blocks can be combined in any order and/or in parallel to implement the process. For discussion purposes, the processes are described with reference to the measuring device 100 shown in Figure 1.

At block 1402, the measuring device 100 may measure a circumference of the limb at a repeatable home location over a period of time as an indicator of interstitial fluid volume in the limb. In some embodiments, the measuring device may be applied to a limb of a human subject and allowed to move to settle at the repeatable home location on the limb. In one or more embodiments, the repeatable home location may be the minimum circumference of the limb, where the strap tension balances the interstitial fluid pressure to enable the device to perform the measurements described above.

At block 1404, the measuring device 100 may generate a waveform showing current circumference data derived from the measured circumference over time. in some embodiments, the circumference measurements may be taken periodically (e.g., once per day), intermittently (e.g., initiated manually), or continuously. In some embodiments, some or all of the data processing and waveform generation may be performed off-device, such as by a remote computing device.

At block 1406, the measuring device may compare the waveform with a waveform of baseline circumference data for the subject at the repeatable home location. The difference in waveforms may be interpreted by the measuring device 105, transmitted to an off-device or remote location, such as a doctor's office, mobile device, portable computer, and/or the like, or interpreted by a human.

At block 1408, the measuring device 105 may output a result of the comparing with an indication exposed by the comparing. For example, if the waveform of current circumference measurements shows an increase over the subject's baseline waveform, an alert may be displayed on the measurement assembly, transmitted to a mobile device or more remote endpoint. Additionally, or in the alternative, an instruction may be output to, e.g., advise the subject of changes to be made in diet or exercise, to revisit or make specific adjustments to treatment (including medicine dosage adjustments), and/or the like.

Although the subject matter has been described in language specific to structural features and/or methodological acts, it is to be understood that the subject matter defined in the appended claims is not necessarily limited to the specific features or acts described above. Rather, the specific features and acts described above are disclosed as an example forms of impending the claims.

## Claims

1. A method, comprising:
applying a measuring device to a limb of a human subject, the measuring device having a strap, a measurement assembly, and a winder cassette;
allowing, in the applying and a subsequent movement by the limb, the measuring device to move under the influence of gravity and/or subject activity to settle at a repeatable home location on the limb, where there is a balance of forces among gravity, interstitial fluid pressure, and tension of the measuring device at the site, wherein the tension is less than or substantially equal to the interstitial fluid pressure;
measuring, via the measuring device, a circumference of the subject's limb at the repeatable home location over a period of time as an indicator of interstitial fluid volume in the limb;
generating a waveform showing current circumference data derived from the measured circumference over time;
comparing the waveform with a waveform of baseline circumference data for the subject at the repeatable home location; and
outputting a result of the comparing with an indication exposed by the comparing;
wherein the repeatable home location is the minimum circumference around the limb at the time of measurement.

2. The method of claim 1, further comprising:
detecting limb orientation during the period of time associated with the circumference reading; and
modulating the waveform to account for the detected limb orientation according to an algorithm.

3. The method of either of claims 1 or 2, further comprising:
illuminating, by an optical emitter, a gradient associated with the strap; and
receiving, by an optical detector, light reflected from the gradient;
wherein the measuring of the circumference includes determining a distance of extension or retraction of the strap around the limb in accordance with the light reflected from the gradient.

4. The method of claim 3, further comprising:
continuously measuring position measurements on the limb by the measuring device;
establishing a relationship between the circumference positions of measurement and position on the gradient where each measurement took place; and
storing in a memory of the measuring device the circumference positions in association with the positions on the gradient;
wherein the measured circumferences are one of determined and stored absolutely or relative to a reference circumference identified for the subject.

5. The method of either of claims 1 or 2, further comprising:
illuminating a first gradient on the strap that gets darker in one direction and a second gradient on the strap that gets lighter in the same direction with respective optical sensors;
detecting amplitudes of light reflected from corresponding locations of the first and second gradients by the optical sensors; and
generating a complementary set of data based on the detected amplitudes such that errors related to nonlinearity of the optical sensors are offset;
wherein the measuring of the circumference includes determining a distance of extension or retraction of the strap around the limb in accordance with the light reflected from the first and second gradients.

6. One or more non-transitory computer-readable media containing instructions that, when executed by one or more processors of a device according to claims 9-15, cause the processors to perform operations, comprising:
continuously measuring, via the measuring device, a circumference of the subject's limb at a repeatable home location over a period of time as an indicator of fluid volume in the limb, the circumference indicating a measure of interstitial fluid volume;
generating a waveform showing current circumference data derived from the measured circumference over time;
detecting limb orientation indicating motion of the subject or lack thereof during the period of time;
modulating the waveform to account for the detected limb orientation according to an algorithm;
comparing the waveform with a waveform of baseline circumference data for the subject at the repeatable home location; and
outputting a result of the comparing with an indication of any health or medical concern exposed by the comparing.

7. The one or more non-transitory computer-readable media of claim 6, the operations further comprising:
detecting, via the measuring device, movement of the limb;
wherein the detected subject activity data indicates motion of the subject from the detected movement of the limb, and
wherein the movement of the limb includes flexing of the Achilles tendon.

8. The one or more non-transitory computer-readable media of either of claims 6 or 7, the operations further comprising:
illuminating, by an optical emitter, a gradient associated with the strap;
receiving, by an optical detector, light reflected from the gradient;
continuously measuring position measurements on the limb by the measuring device;
establishing a relationship between the circumference positions of measurement and position on the gradient at where each measurement took place; and
storing in the memory the circumference positions in association with the positions on the gradient;
wherein the measuring of the circumference includes determining a distance of extension or retraction of the strap around the limb in accordance with the light reflected from the gradient;
wherein the measured circumferences are one of determined and stored absolutely or relative to a reference circumference identified for the subject; and
wherein the repeatable home location is the minimum circumference around the limb at the time of measurement.

9. A device, comprising:
a winder cassette (600) comprising a clasp portion (615) and a spindle assembly (625), wherein:
the clasp portion is configured to be attached to the spindle assembly and to a first strap end, and comprises a first latch feature (620) configured to be latched to the spindle assembly; and
the spindle assembly comprises:
a hollow spindle;
a spring (670) located at least partially inside the spindle, operably coupled to
the spindle, and attached to a second strap end; and a second latch feature (620) configured to be latched to the first latch feature of the clasp portion; and
a measurement assembly wherein the measurement assembly comprises an electronics subassembly (420) and an enclosure, wherein the electronics subassembly includes:
measurement components including one or more optical sensors (520), one or more processors, memory, and executable instructions stored in the memory that, if executed by the one or more processors, cause the one or more processors to perform operations comprising:
measuring a circumference of a subject's limb at a repeatable home location over a period of time as an indicator of interstitial fluid volume in the limb;
generating a waveform showing current circumference data derived from the measured circumference over time;
comparing the waveform with a waveform of baseline circumference data for the subject at the repeatable home location; and
outputting a result of the comparing with an indication exposed by the comparing.

10. The device of claim 9, wherein the operations further comprise:
detecting limb orientation during the period of time associated with the circumference reading; and
modulating the waveform to account for the detected limb orientation according to an algorithm.

11. The device of either of claims 9 or 10, wherein the measurement components comprise optical sensors that are positioned to face the strap, emit light to the strap, detect light reflected from the strap, and output a signal corresponding to the amount of the detected light; and
wherein the operations further comprise:
comparing the amount of detected light from the output signal with data associating detected light with limb circumference; and
determining the current circumference at the repeatable home location in accordance with the comparing;
wherein the optical sensors are arranged to:
detect amplitudes of light reflected from corresponding locations of a first gradient on the strap that gets darker in one direction and a second gradient on the strap that gets lighter in the same direction; and
generate a complementary set of data based on the detected amplitudes from the respective optical sensors such that errors related to nonlinearity of the two optical sensors are offset;
or wherein the optical sensors are arranged to:
detect amplitudes of light reflected from corresponding locations of a single gradient on the strap that gets darker in one direction; and
generate a complementary set of data based on the detected amplitudes from the respective optical sensors such that errors related to nonlinearity of the two optical sensors are offset;
wherein the repeatable home location is at the minimum circumference of the limb.

12. The device of either of claims 9 or 11, wherein the operations further comprise:
receiving data related to a swelling state of the limb at the repeatable home location;
determining at least one pattern of limb swelling changes over time in the received data;
determining, from the at least one pattern, one or more of stable, transient, or trending changes of swelling associated with an impending heart failure decompensation;
determining a level of a condition of an impending heart failure decompensation that exceeds a predetermined threshold; and
outputting instructions related to the subject's condition.

13. The device of any of claims 9, **11** and 12, wherein the operations further comprise:
determining changes in viscosity of interstitial fluid in the subject based on the circumference measurements.

14. The device of any of claims 9-13, wherein the spindle assembly further comprises a capture feature (630) configured to operably couple the spring to the spindle; and
wherein the capture feature comprises a bar that extends from an interior wall of the spindle and includes a gap configured for a first end of the spring to pass through the gap and wrap at least part way around the bar to anchor the spring to the spindle.

15. The device of any of claims 9-13, wherein the spindle assembly further comprises a capture feature (630) configured to operably couple the spring to the spindle; and
wherein the capture feature includes first and second pin portions spaced apart by a gap configured for a first end of the spring to pass through the gap and wrap at least part way around at least one of the first and second pin portions to anchor the spring to the spindle.

## Patentansprüche

1. Verfahren, umfassend:
Anwenden einer Messvorrichtung an einer Extremität eines menschlichen Subjekts, wobei die Messvorrichtung ein Band, eine Messanordnung und eine Wickelkassette aufweist;
Ermöglichen, dass sich die Messvorrichtung, bei der Anwendung und einer anschließenden Bewegung durch die Extremität, unter dem Einfluss von Schwerkraft und/oder Aktivität des Subjekts bewegt, um sich an einer wiederholbaren Ausgangsstellung an der Extremität einzustellen, an der ein Kräftegleichgewicht zwischen Schwerkraft, interstitiellem Flüssigkeitsdruck und Spannung der Messvorrichtung an der Stelle besteht, wobei die Spannung weniger als oder im Wesentlichen gleich dem interstitiellen Flüssigkeitsdruck ist;
Messen, über die Messvorrichtung, eines Umfangs der Extremität des Subjekts an der wiederholbaren Ausgangsstellung über einen Zeitraum als ein Indikator für interstitielles Flüssigkeitsvolumen in der Extremität;
Generieren einer Wellenform, die aktuelle Umfangsdaten zeigt, die von dem gemessenen Umfang über die Zeit abgeleitet sind;
Vergleichen der Wellenform mit einer Wellenform von Basislinien-Umfangsdaten für das Subjekt an der wiederholbaren Ausgangsstellung; und
Ausgeben eines Ergebnisses des Vergleichens mit einer durch das Vergleichen aufgedeckten Angabe;
wobei die wiederholbare Ausgangsstellung der minimale Umfang um die Extremität zum Zeitpunkt der Messung ist.

2. Verfahren nach Anspruch 1, ferner umfassend:
Erfassen von Extremitätsausrichtung während des Zeitraums, der dem Umfangslesen zugeordnet ist; und
Modulieren der Wellenform, um die erfasste Extremitätsausrichtung gemäß einem Algorithmus zu berücksichtigen.

3. Verfahren nach einem von Anspruch 1 oder 2, ferner umfassend:
Beleuchten eines dem Band zugeordneten Gradienten durch einen optischen Sender; und
Empfangen, durch einen optischen Detektor, von Licht, das von dem Gradienten reflektiert wird;
wobei das Messen des Umfangs das Bestimmen einer Strecke eines Ausfahrens oder Zurückziehens des Bandes um die Extremität gemäß dem von dem Gradienten reflektierten Licht beinhaltet.

4. Verfahren nach Anspruch 3, ferner umfassend:
kontinuierliches Messen von Positionsmessungen an der Extremität durch die Messvorrichtung;
Herstellen einer Beziehung zwischen den Umfangsmesspositionen und der Position auf dem Gradienten, an dem jede Messung stattgefunden hat; und
Speichern, in einem Speicher der Messvorrichtung, der Umfangspositionen in Verbindung mit den Positionen auf dem Gradienten;
wobei die gemessenen Umfänge eines von bestimmt und absolut oder relativ zu einem für das Subjekt identifizierten Referenzumfang gespeichert sind.

5. Verfahren nach einem von Anspruch 1 oder 2, ferner umfassend:
Beleuchten eines ersten Gradienten an dem Band, der in einer Richtung dunkler wird, und eines zweiten Gradienten an dem Band, der in der gleichen Richtung heller wird, mit jeweiligen optischen Sensoren;
Erfassen von Amplituden von Licht, das von entsprechenden Stellen des ersten und des zweiten Gradienten reflektiert wird, durch die optischen Sensoren; und
Generieren eines komplementären Datensatzes basierend auf den erfassten Amplituden, sodass Fehler, die sich auf Nichtlinearität der optischen Sensoren beziehen, ausgeglichen werden;
wobei das Messen des Umfangs das Bestimmen einer Strecke des Ausfahrens oder Zurückziehens des Bandes um die Extremität gemäß dem von dem ersten und dem zweiten Gradienten reflektierten Licht beinhaltet.

6. Ein oder mehrere nichtflüchtige computerlesbare Medien, enthaltend Anweisungen, die, wenn sie durch einen oder mehrere Prozessoren einer Vorrichtung nach Anspruch 9-15 ausgeführt werden, die Prozessoren veranlassen, Operationen durchführen, die Folgendes umfassen:
kontinuierliches Messen, über die Messvorrichtung, eines Umfangs der Extremität des Subjekts an einer wiederholbaren Ausgangsstellung über einen Zeitraum als ein Indikator für Flüssigkeitsvolumen in der Extremität, wobei der Umfang ein Maß für interstitielles Flüssigkeitsvolumen angibt;
Generieren einer Wellenform, die aktuelle Umfangsdaten zeigt, die von dem gemessenen Umfang über die Zeit abgeleitet sind;
Erfassen von Extremitätsausrichtung, die eine Bewegung des Subjekts oder ein Fehlen davon während des Zeitraums angibt;
Modulieren der Wellenform, um die erfasste Extremitätsausrichtung gemäß einem Algorithmus zu berücksichtigen;
Vergleichen der Wellenform mit einer Wellenform von Basislinien-Umfangsdaten für das Subjekt an der wiederholbaren Ausgangsstellung; und
Ausgeben eines Ergebnisses des Vergleichens mit einer durch das Vergleichen aufgedeckten Angabe eines gesundheitlichen oder medizinischen Problems.

7. Ein oder mehrere nichtflüchtige computerlesbare Medien nach Anspruch 6, wobei die Operationen ferner Folgendes umfassen:
Erfassen, über die Messvorrichtung, von Bewegung der Extremität;
wobei die erfassten Subjektsaktivitätsdaten eine Bewegung des Subjekts aus der erfassten Bewegung der Extremität angeben und
wobei die Bewegung der Extremität das Beugen der Achillessehne beinhaltet.

8. Ein oder mehrere nichtflüchtige computerlesbare Medien nach einem von Anspruch 6 oder 7, wobei die Operationen ferner Folgendes umfassen:
Beleuchten eines dem Band zugeordneten Gradienten durch einen optischen Sender;
Empfangen, durch einen optischen Detektor, von Licht, das von dem Gradienten reflektiert wird;
kontinuierliches Messen von Positionsmessungen an der Extremität durch die Messvorrichtung;
Herstellen einer Beziehung zwischen den Umfangsmesspositionen und der Position auf dem Gradienten, an dem jede Messung stattgefunden hat; und
Speichern, in dem Speicher, der Umfangspositionen in Verbindung mit den Positionen auf dem Gradienten;
wobei das Messen des Umfangs Bestimmen einer Strecke des Ausfahrens oder Zurückziehens des Bandes um die Extremität gemäß dem von dem Gradienten reflektierten Licht beinhaltet;
wobei die gemessenen Umfänge eines von bestimmt und absolut oder relativ zu einem für das Subjekt identifizierten Referenzumfang gespeichert sind; und
wobei die wiederholbare Ausgangsstellung der minimale Umfang um die Extremität zum Zeitpunkt der Messung ist.

9. Vorrichtung, umfassend:
eine Wickelkassette (600), die einen Klammerabschnitt (615) und eine Spindelanordnung (625) umfasst, wobei:
der Klammerabschnitt dazu konfiguriert ist, an der Spindelanordnung und an einem ersten Bandende befestigt zu werden, und ein erstes Verriegelungsmerkmal (620) umfasst, das dazu konfiguriert ist, an der Spindelanordnung verriegelt zu werden; und
die Spindelanordnung Folgendes umfasst:
eine Hohlspindel;
eine Feder (670), die sich zumindest teilweise innerhalb der Spindel befindet, mit der Spindel wirkverbunden ist und an einem zweiten Bandende befestigt ist; und
ein zweites Verriegelungsmerkmal (620), das dazu konfiguriert ist, mit dem ersten Verriegelungsmerkmal des Klammerabschnitts verriegelt zu werden; und
eine Messanordnung, wobei die Messanordnung eine Elektronik-Unteranordnung (420) und ein Gehäuse umfasst, wobei die Elektronik-Unteranordnung Folgendes beinhaltet:
Messkomponenten, beinhaltend einen oder mehrere optische Sensoren (520), ein oder mehrere Prozessoren, Speicher und ausführbare Anweisungen, die in dem Speicher gespeichert sind und die, wenn sie durch den einen oder die mehreren Prozessoren ausgeführt werden, den einen oder die mehreren Prozessoren veranlassen, Operationen durchzuführen, die Folgendes umfassen:
Messen eines Umfangs einer Extremität eines Subjekts an einer wiederholbaren Ausgangsstellung über einen Zeitraum als ein Indikator für interstitielles Flüssigkeitsvolumen in der Extremität;
Generieren einer Wellenform, die aktuelle Umfangsdaten zeigt, die von dem gemessenen Umfang über die Zeit abgeleitet sind;
Vergleichen der Wellenform mit einer Wellenform von Basislinien-Umfangsdaten für das Subjekt an der wiederholbaren Ausgangsstellung; und
Ausgeben eines Ergebnisses des Vergleichens mit einer durch das Vergleichen aufgedeckten Angabe.

10. Vorrichtung nach Anspruch 9, wobei die Operationen ferner Folgendes umfassen:
Erfassen von Extremitätsausrichtung während des Zeitraums, der dem Umfangslesen zugeordnet ist; und
Modulieren der Wellenform, um die erfasste Extremitätsausrichtung gemäß einem Algorithmus zu berücksichtigen.

11. Vorrichtung nach einem von Anspruch 9 oder 10, wobei die Messkomponenten optische Sensoren umfassen, die so positioniert sind, dass sie dem Band zugewandt sind, Licht an das Band senden, von dem Band reflektiertes Licht erfassen und ein Signal ausgeben, das der Menge des erfassten Lichts entspricht; und
wobei die Operationen ferner Folgendes umfassen:
Vergleichen der Menge an erfasstem Licht aus dem Ausgangssignal mit Daten, die erfasstes Licht einem Extremitätsumfang zuordnen; und
Bestimmen des aktuellen Umfangs an der wiederholbaren Ausgangsstellung gemäß dem Vergleichen;
wobei die optischen Sensoren zu Folgendem angeordnet sind:
Erfassen von Amplituden von Licht, das von entsprechenden Stellen eines ersten Gradienten an dem Band, der in einer Richtung dunkler wird, und eines zweiten Gradienten an dem Band, der in der gleichen Richtung heller wird, reflektiert wird; und
Generieren eines komplementären Datensatzes basierend auf den erfassten Amplituden von den jeweiligen optischen Sensoren, sodass Fehler, die sich auf Nichtlinearität der beiden optischen Sensoren beziehen, ausgeglichen werden;
oder wobei die optischen Sensoren zu Folgendem angeordnet sind:
Erfassen von Amplituden von Licht, das von entsprechenden Stellen eines einzelnen Gradienten an dem Band , der in einer Richtung dunkler wird, reflektiert wird; und
Generieren eines komplementären Datensatzes basierend auf den erfassten Amplituden von den jeweiligen optischen Sensoren, sodass Fehler, die sich auf Nichtlinearität der beiden optischen Sensoren beziehen, ausgeglichen werden;
wobei sich die wiederholbare Ausgangsstellung am minimalen Umfang der Extremität befindet.

12. Vorrichtung nach einem von Anspruch 9 oder 11, wobei die Operationen ferner Folgendes umfassen:
Empfangen von Daten, die sich auf einen Schwellungszustand der Extremität an der wiederholbaren Ausgangsstellung beziehen;
Bestimmen von mindestens einem Muster von Extremitätsschwellungsänderungen über die Zeit in den empfangenen Daten;
Bestimmen, aus dem mindestens einen Muster, von einer oder mehreren von stabilen, vorübergehenden oder tendenziellen Änderungen von Schwellungen, die einer bevorstehenden Herzinsuffizienzdekompensation zugeordnet sind;
Bestimmen eines Niveaus eines Zustands einer bevorstehenden Herzinsuffizienzdekompensation, der einen vorbestimmten Schwellenwert überschreitet; und
Ausgeben von Anweisungen, die sich auf den Zustand des Subjekts beziehen.

13. Vorrichtung nach einem der Ansprüche 9, 11 und 12, wobei die Operationen ferner Folgendes umfassen:
Bestimmen von Änderungen einer Viskosität von interstitieller Flüssigkeit in dem Subjekt basierend auf den Umfangsmessungen.

14. Vorrichtung nach einem der Ansprüche 9-13, wobei die Spindelanordnung ferner ein Einfangmerkmal (630) umfasst, das dazu konfiguriert ist, die Feder wirksam an die Spindel zu koppeln; und
wobei das Einfangmerkmal einen Stab umfasst, der sich von einer Innenwand der Spindel erstreckt und einen Spalt beinhaltet, der dafür konfiguriert ist, dass ein erstes Ende der Feder durch den Spalt verläuft und sich zumindest teilweise um den Stab wickelt, um die Feder an der Spindel zu verankern.

15. Vorrichtung nach einem der Ansprüche 9-13, wobei die Spindelanordnung ferner ein Einfangmerkmal (630) umfasst, das dazu konfiguriert ist, die Feder wirksam an die Spindel zu koppeln; und
wobei das Einfangmerkmal einen ersten und einen zweiten Stiftabschnitt beinhaltet, die durch einen Spalt voneinander beabstandet sind, der dafür konfiguriert ist, dass ein erstes Ende der Feder durch den Spalt verläuft und sich zumindest teilweise um zumindest einen von dem ersten und dem zweiten Stiftabschnitt wickelt, um die Feder an der Spindel zu verankern.

## Revendications

1. Procédé, comprenant :
l'application d'un dispositif de mesure sur un membre d'un sujet humain, le dispositif de mesure comportant une sangle, un ensemble de mesure et une cassette enrouleuse ;
le fait de permettre, lors de l'application et d'un mouvement ultérieur du membre, au dispositif de mesure de se déplacer sous l'effet de la gravité et/ou de l'activité du sujet de manière à s'installer à un emplacement d'origine reproductible sur le membre, où il existe un équilibre des forces entre la gravité, la pression du fluide interstitiel et la tension du dispositif de mesure sur le site, ladite tension étant inférieure ou sensiblement égale à la pression du fluide interstitiel ;
la mesure, par l'intermédiaire du dispositif de mesure, de la circonférence du membre du sujet au niveau de l'emplacement d'origine reproductible sur une période de temps en tant qu'indicateur du volume de fluide interstitiel dans le membre ;
la génération d'une forme d'onde montrant des données de circonférence courante dérivées de la circonférence mesurée au fil du temps ;
la comparaison de la forme d'onde avec une forme d'onde de données de circonférence de base pour le sujet à l'emplacement d'origine reproductible ; et
la sortie d'un résultat de la comparaison avec une indication exposée par la comparaison ;
ledit emplacement d'origine reproductible étant la circonférence minimale autour du membre au moment de la mesure.

2. Procédé selon la revendication 1, comprenant en outre :
la détection de l'orientation du membre pendant la période de temps associée à la lecture de la circonférence ; et
la modulation de la forme d'onde pour tenir compte de l'orientation du membre détectée conformément à un algorithme.

3. Procédé selon l'une quelconque des revendications 1 ou 2, comprenant en outre :
l'éclairage, par un émetteur optique, d'un gradient associé à la bande ; et
la réception, par un détecteur optique, de la lumière réfléchie par le gradient ;
ladite mesure de la circonférence comprenant la détermination de la distance d'extension ou de rétraction de la sangle autour du membre en fonction de la lumière réfléchie par le gradient.

4. Procédé selon la revendication 3, comprenant en outre :
la mesure continue des mesures de position sur le membre par le dispositif de mesure ;
l'établissement d'une relation entre les positions circonférentielles de mesure et la position sur le gradient où chaque mesure a eu lieu ; et
le stockage dans une mémoire du dispositif de mesure des positions de circonférence en association avec les positions sur le gradient ;
lesdites circonférences mesurées étant l'une des circonférences déterminées et stockées absolument ou par rapport à une circonférence de référence identifiée pour le sujet.

5. Procédé selon l'une quelconque des revendications 1 ou 2, comprenant en outre :
l'éclairage d'un premier gradient sur la sangle qui devient plus sombre suivant une direction et un second gradient sur la sangle qui devient plus clair suivant la même direction avec des capteurs optiques respectifs ;
la détection des amplitudes de lumière réfléchie à partir des emplacements correspondants des premier et second gradients par les capteurs optiques ; et
la génération d'un ensemble complémentaire de données sur la base des amplitudes détectées de sorte que les erreurs liées à la non-linéarité des capteurs optiques soient décalées ;
ladite mesure de la circonférence comprenant la détermination de la distance d'extension ou de rétraction de la sangle autour du membre en fonction de la lumière réfléchie par les premier et second gradients.

6. Un ou plusieurs supports non transitoires lisibles par ordinateur contenant des instructions qui, lorsqu'elles sont exécutées par un ou plusieurs processeurs d'un dispositif selon les revendications 9 à 15, amènent les processeurs à réaliser des opérations, comprenant :
la mesure en continu, par l'intermédiaire du dispositif de mesure, de la circonférence du membre du sujet au niveau d'un emplacement d'origine reproductible sur une période de temps en tant qu'indicateur du volume de fluide dans le membre, la circonférence indiquant une mesure du volume de fluide interstitiel ;
la génération d'une forme d'onde montrant des données de circonférence courante dérivées de la circonférence mesurée au fil du temps ;
la détection de l'orientation du membre indiquant le mouvement du sujet ou l'absence de celui-ci pendant la période de temps ;
la modulation de la forme d'onde pour tenir compte de l'orientation du membre détectée conformément à un algorithme ;
la comparaison de la forme d'onde avec une forme d'onde de données de circonférence de base pour le sujet à l'emplacement d'origine reproductible ; et
la sortie d'un résultat de la comparaison avec une indication de tout problème de santé ou médical exposé par la comparaison.

7. Un ou plusieurs supports non transitoires lisibles par ordinateur selon la revendication 6, les opérations comprenant en outre :
la détection, par l'intermédiaire du dispositif de mesure, du mouvement du membre ;
lesdites données d'activité du sujet détectées indiquant le mouvement du sujet à partir du mouvement détecté du membre, et
ledit mouvement du membre comprenant la flexion du tendon d'Achille.

8. Un ou plusieurs supports non transitoires lisibles par ordinateur selon l'une quelconque des revendications 6 ou 7, les opérations comprenant en outre :
l'éclairage, par un émetteur optique, d'un gradient associé à la sangle ;
la réception, par un détecteur optique, de la lumière réfléchie par le gradient ;
la mesure continue des mesures de position sur le membre par le dispositif de mesure ;
l'établissement d'une relation entre les positions circonférentielles de mesure et la position sur le gradient à l'endroit où chaque mesure a eu lieu ; et
le stockage dans la mémoire des positions circonférentielles en association avec les positions sur le gradient ;
ladite mesure de la circonférence comprenant la détermination de la distance d'extension ou de rétraction de la sangle autour du membre en fonction de la lumière réfléchie par le gradient ;
lesdites circonférences mesurées étant l'une des circonférences déterminées et stockées absolument ou par rapport à une circonférence de référence identifiée pour le sujet ; et
ledit emplacement d'origine reproductible étant la circonférence minimale autour du membre au moment de la mesure.

9. Dispositif, comprenant :
une cassette enrouleuse (600) comprenant une partie fermoir (615) et un ensemble broche (625) :
ladite partie fermoir étant conçue pour être fixée à l'ensemble broche et à une première extrémité de sangle, et comprenant un premier élément de verrouillage (620) conçu pour être verrouillé à l'ensemble broche ; et
ledit ensemble broche comprenant :
une broche creuse ;
un ressort (670) situé au moins partiellement à l'intérieur de la broche, couplé fonctionnellement à la broche et fixé à une seconde extrémité de sangle ; et
un second élément de verrouillage (620) conçu pour être verrouillé au premier élément de verrouillage de la partie fermoir ; et
un ensemble de mesure, ledit ensemble de mesure comprenant un sous-ensemble électronique (420) et une enceinte, ledit sous-ensemble électronique comprenant :
des composants de mesure comprenant un ou plusieurs capteurs optiques (520), un ou plusieurs processeurs, une mémoire et des instructions exécutables stockées dans la mémoire qui, si elles sont exécutées par lesdits un ou plusieurs processeurs, amènent lesdits un ou plusieurs processeurs à réaliser des opérations comprenant :
la mesure de la circonférence du membre d'un sujet au niveau d'un emplacement d'origine reproductible sur une période de temps en tant qu'indicateur du volume de fluide interstitiel dans le membre ;
la génération d'une forme d'onde montrant des données de circonférence courante dérivées de la circonférence mesurée au fil du temps ;
la comparaison de la forme d'onde avec une forme d'onde de données de circonférence de base pour le sujet à l'emplacement d'origine reproductible ; et
la sortie d'un résultat de la comparaison avec une indication exposée par la comparaison.

10. Dispositif selon la revendication 9, lesdites opérations comprenant en outre :
la détection de l'orientation du membre pendant la période de temps associée à la lecture de la circonférence ; et
la modulation de la forme d'onde pour tenir compte de l'orientation du membre détectée conformément à un algorithme.

11. Dispositif selon l'une quelconque des revendications 9 ou 10, lesdits composants de mesure comprenant des capteurs optiques qui sont positionnés de manière à faire face à la bande, émettre de la lumière vers la bande, détecter la lumière réfléchie par la bande et émettre un signal correspondant à la quantité de lumière détectée ; et
lesdites opérations comprenant en outre :
la comparaison de la quantité de lumière détectée à partir du signal de sortie avec des données associant la lumière détectée à la circonférence du membre ; et
la détermination de la circonférence courante à l'emplacement d'origine reproductible conformément à la comparaison ;
lesdits capteurs optiques étant agencés pour :
détecter des amplitudes de lumière réfléchies à partir d'emplacements correspondants d'un premier gradient sur la sangle qui devient plus sombre suivant une direction et un second gradient sur la sangle qui devient plus clair suivant la même direction ; et
générer un ensemble complémentaire de données sur la base des amplitudes détectées à partir des capteurs optiques respectifs de sorte que les erreurs liées à la non-linéarité des deux capteurs optiques soient décalées ;
ou lesdits capteurs optiques étant agencés de manière à :
détecter des amplitudes de lumière réfléchie à partir des emplacements correspondants d'un seul gradient sur la sangle qui devient plus sombre suivant une direction ; et
générer un ensemble complémentaire de données sur la base des amplitudes détectées à partir des capteurs optiques respectifs de sorte que les erreurs liées à la non-linéarité des deux capteurs optiques soient décalées ;
ledit emplacement d'origine reproductible se trouvant à la circonférence minimale du membre.

12. Dispositif selon l'une quelconque des revendications 9 ou 11, lesdites opérations comprenant en outre :
la réception de données relatives à un état de gonflement du membre au niveau de l'emplacement d'origine reproductible ;
la détermination d'au moins un modèle de changements de gonflement du membre au fil du temps dans les données reçues ;
la détermination, à partir dudit au moins un modèle, d'un ou plusieurs changements stables, transitoires ou tendanciels de gonflement associés à une décompensation imminente d'insuffisance cardiaque ;
la détermination du niveau d'un état d'une décompensation imminente d'insuffisance cardiaque qui dépasse un seuil prédéfini ; et
la sortie d'instructions liées à l'état du sujet.

13. Dispositif selon l'une quelconque des revendications 9, 11 et 12, lesdites opérations comprenant en outre :
la détermination des changements de viscosité du fluide interstitiel chez le sujet sur la base des mesures de la circonférence.

14. Dispositif selon l'une quelconque des revendications 9 à 13, ledit ensemble broche comprenant en outre un élément de capture (630) conçu pour coupler de manière fonctionnelle le ressort à la broche ; et
ledit élément de capture comprenant une barre qui s'étend à partir d'une paroi intérieure de la broche et comprenant un espace conçu pour qu'une première extrémité du ressort passe à travers l'espace et s'enroule au moins en partie autour de la barre de manière à ancrer le ressort à la broche.

15. Dispositif selon l'une quelconque des revendications 9 à 13, ledit ensemble broche comprenant en outre un élément de capture (630) conçu pour coupler de manière fonctionnelle le ressort à la broche ; et
ledit élément de capture comprenant des première et seconde parties de tige espacées par un espace conçu pour qu'une première extrémité du ressort passe à travers l'espace et s'enroule au moins partiellement autour d'au moins l'une des première et seconde parties de tige de manière à ancrer le ressort à la broche.
